# EUROPÄISCHE PATENTSCHRIFT

(11) **EP 4 417 181 B1**
(45) Veröffentlichungstag und Bekanntmachung des Hinweises auf die Patenterteilung: **15.04.2026**
(21) Anmeldenummer: 23218014.1
(22) Anmeldetag: 19.12.2023
(51) Int. Cl.: A61K 8/02, A61K 8/37, A61K 8/92, A61Q 1/00, A61Q 1/02

(54) **MINERALÖLFREIER LIPPENSTIFT MIT HOHEM GLYCERINGEHALT**
MINERAL OIL FREE HIGH GLYCERIN LIPSTICK
ROUGE À LÈVRES SANS HUILE MINÉRALE ET À TENEUR ÉLEVÉE EN GLYCÉRINE

(30) Priorität: 19.01.2023 DE 102023200406
(43) Veröffentlichungstag der Anmeldung: 21.08.2024
(73) Patentinhaber: Beiersdorf AG, 22529 Hamburg (DE)
(72) Erfinder: Rathsack, Jessica, 21614 Buxtehude (DE); Hartwig, Tanja, 23843 Bad Oldeslohe (DE); Fiedler, Dorothe, 22335 Hamburg (DE)

(56) Entgegenhaltungen:
- WO-A1-2012/016642
- WO-A1-2021/239384
- CN-A- 111 481 475
- DATABASE GNPD [online] MINTEL; 5 March 2019 (2019-03-05), ANONYMOUS: "Moisture & Relief Rich Lip Cream", XP093172864, retrieved from https://www.gnpd.com/sinatra/recordpage/6379791/ Database accession no. 6379791

## Beschreibung

Die vorliegende Erfindung betrifft einen kosmetischen Lippenstift auf der Basis einer Glycerin-in-Öl-Emulsion enthaltend
a) Diisostearoyl Polyglyceryl-3 Dimer Dilinoleate,
b) einem zweiten Emulgator aus der Gruppe der Verbindungen Polyglyceryl-4 Diisostearate/Polyhydroxystearate/Sebacate,
c) Sonnenblumenwachs, wobei die Zubereitung frei ist von Mineralöl, Paraffinwachs, mikrokristallinem Wachs, Schellackwachs, Polyethylenwachsen, Polyacrylaten und quervernetzten Acrylat/C10-C30 Alkylacrylat Polymeren, dadurch gekennzeichnet, dass die Zubereitung Carnaubawachs enthält, sowie dessen Herstellungsverfahren.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Neben Gesichtspudern und Rouge als pulverförmigen Kosmetika sowie stiftförmigen Zubereitungen werden hierzu cremeförmige Präparate, wie Tagescremes und Creme-Make-up auf Emulsionsbasis verwendet.

Zu den am meisten verwendeten dekorativen Kosmetika gehört der Lippenstift. Mehr als 50 % der Frauen in Deutschland benutzen ihn. Meist dient er neben der farblichen Betonung der Lippen auch zur Lippenpflege.

Lippenstifte bestehen in der Regel aus einer Wachsmatrix, in die in geringer Konzentration flüssige und halbfeste Öle sowie Pigmente und Füllstoffe eingearbeitet sind. Sie liegen in fester Stiftform vor.

Nachteilig am Stande der Technik ist der Umstand, dass Lippenpflegeprodukte wie Lippencremes, Lippenbutter etc. und insbesondere die Lippenstifte des Standes der Technik häufig Mineralöle und Paraffinwachse enthalten, die bei den Verbrauchern zunehmend unbeliebt sind. Neben einem generellen Wunsch nach Produkten aus nachwachsenden Rohstoffen, spielt dabei auch die Sorge vor gesundheitsgefährdenden Verunreinigungen, die in den auf Rohölbasis hergestellten Rohstoffen enthalten sein könnten, eine Rolle. Ob diese Sorge tatsächlich berechtigt ist, kann im Rahmen der vorliegenden Offenbarung dahingestellt bleiben. Tatsache ist, dass bei vielen Verbrauchern der Wunsch nach "mineralölfreien" Produkten besteht.

Es war daher die Aufgabe der vorliegenden Erfindung, einen "mineralölfreien" Lippenstift zu entwickeln.

Um die Pflegeeigenschaften von Lippenstiften zu verbessern wird den Rezepturen häufig Glycerin zugesetzt. Glycerin dient dabei der Befeuchtung der Lippen und sorgt dafür, dass die Lippen nicht austrocknen und rissig werden.

Bei der Herstellung mineralölfreier bzw. Paraffinwachs freier Lippenstifte mit höheren Glycerin-Gehalten (größer 20 Gewichts-%) kommt es bei der Herstellung jedoch zu ungeahnten Problemen:
Nachdem die heiße Stiftmasse in die Stiftform gefüllt wurde, muss sie abkühlen, um fest zu werden und sich als Stift aus der Form herauslösen zu lassen. Während der Herstellung nimmt die durch das Glycerin hygroskopisch gewordene Stiftmasse jedoch aus der Umgebungsluft Wasser auf, was dazu führt, dass die Stiftmasse beim Abkühlen nicht mehr richtig aushärtet, sondern weicher und klebriger wird und sich nicht mehr aus der Stiftform herauslösen (entformen) lässt. Vielmehr bleibt sie an der Form kleben.

Es war daher die Aufgabe der vorliegenden Erfindung, diesen Nachteil des Standes der Technik zu beseitigen und einen mineralölfreien und Paraffinwachs- freien Lippenstift mit höherem Glycerin-Gehalt (größer 20 Gewichts-%) zu entwickeln, der sich bei der Herstellung beim Abkühlen aushärten und anschließend problemlos aus der Stiftform herauslösen lässt.

Überraschend gelöst wird die Aufgabe durch einen kosmetischen Lippenstift auf der Basis einer Glycerin-in-Öl-Emulsion enthaltend
a) Diisostearoyl Polyglyceryl-3 Dimer Dilinoleate,
b) einem zweiten Emulgator aus der Gruppe der Verbindungen Polyglyceryl-4 Diisostearate/Polyhydroxystearate/Sebacate,
c) Sonnenblumenwachs, wobei die Zubereitung frei ist von Mineralöl, Paraffinwachs, mikrokristallinem Wachs, Schellackwachs, Polyethylenwachsen, Polyacrylaten und quervernetzten Acrylat/C10-C30 Alkylacrylat Polymeren, dadurch gekennzeichnet, dass die Zubereitung Carnaubawachs enthält.

Überraschend gelöst wird die Aufgabe ferner durch Verfahren zur Herstellung eines Lippenstiftes auf der Basis einer Glycerin-in-Öl-Emulsion, wobei die Ölphase
a) Diisostearoyl Polyglyceryl-3 Dimer Dilinoleate
b) Polyglyceryl-4 Diisostearate/Polyhydroxystearate/Sebacate,
c) Sonnenblumenwachs und
d) Carnaubawachs enthält, wobei die Zubereitung frei ist von Mineralöl, Paraffinwachs, mikrokristallinem Wachs, Schellackwachs, Polyethylenwachsen, Polyacrylaten und quervernetzten Acrylat/C10-C30 Alkylacrylat Polymeren, dadurch gekennzeichnet, dass die Glycerinphase und die Ölphase zunächst bei einer Temperatur von jeweils größer/gleich 80 °C vereinigt und homogenisiert werden.

Zwar kennt der Fachmann die WO 2021/239384, doch konnte diese Schrift nicht den Weg zur vorliegenden Erfindung weisen.

Im Rahmen der vorliegenden Offenbarung beziehen sich die Formulierungen "erfindungsgemäß", "erfindungsgemäße Zubereitung" etc. immer auf die erfindungsgemäßen Zubereitungen und Verfahren, d.h. auch auf Zubereitungen, mit denen das erfindungsgemäße Verfahren verwirklicht wird.

Es ist erfindungsgemäß von Vorteil, wenn die Zubereitung Glycerin in einer Menge von 21 bis 30 Gewichts-%, bezogen auf das Gesamtgewicht der Zubereitung, enthält.

Ferner ist es erfindungsgemäß vorteilhaft, wenn die Zubereitung Wasser in einer Konzentration weniger als 6 Gewichts-%, bezogen auf das Gesamtgewicht der Zubereitung, enthält.

Vorteilhafte Ausführungsformen der vorliegenden Erfindung sind außerdem dadurch gekennzeichnet, dass die Zubereitung Diisostearoyl Polyglyceryl-3 Dimer Dilinoleate in einer Konzentration von 1 bis 4 Gewichts-%, bezogen auf das Gesamtgewicht der Zubereitung, enthält.

Darüber hinaus ist es vorteilhaft im Sinne der vorliegenden Erfindung, wenn die Zubereitung Polyglyceryl-4 Diisostearate/Polyhydroxystearate/Sebacate in einer Konzentration von 0,5 bis 2,5 Gewichts-%, bezogen auf das Gesamtgewicht der Zubereitung, enthält.

Ferner ist es erfindungsgemäß von Vorteil, wenn die Zubereitung Sonnenblumenwachs in einer Konzentration von 1,0 bis 10 Gewichts-%, bezogen auf das Gesamtgewicht der Zubereitung, enthält.

Vorteilhafte Ausführungsformen im Sinne der vorliegenden Erfindung sind auch dadurch gekennzeichnet, dass die Zubereitung Carnaubawachs in einer Konzentration von 0,3 bis 2 Gewichts-%, bezogen auf das Gesamtgewicht der Zubereitung, enthält.

Darüber hinaus ist es vorteilhaft im Sinne der vorliegenden Erfindung, wenn die Zubereitung Behenylbehenat (INCI: Behenyl Behenate) enthält.

In einem solchen Falle ist es erfindungsgemäß bevorzugt, wenn die Zubereitung Behenylbehenat (INCI: Behenyl Behenate) in einer Konzentration von 1,0 bis 10 Gewichts-%, bezogen auf das Gesamtgewicht der Zubereitung, enthält.

Außerdem ist es erfindungsgemäß von Vorteil, wenn die Zubereitung Bienenwachs (INCI: Cera alba) enthält.

In einem solchen Falle ist es erfindungsgemäß bevorzugt, wenn die Zubereitung Bienenwachs (INCI: Cera alba) in einer Konzentration von 1 bis 10 Gewichts-%, bezogen auf das Gesamtgewicht der Zubereitung, enthält.

Darüber hinaus kann die Lippenstiftzubereitung die für solche Produkte üblichen Inhaltstoffe enthalten, beispielsweise weitere lipophile Bestandteile, Wirkstoffe wie Coenzym Q10, natürliche und/oder synthetische Isoflavonoide, Flavonoide, Kreatin, Kreatinin, Taurin, β-Alanin, Panthenol, Tocopherylacetat, Thiamidol; Allantoin, Vitamin E bzw. Vitamin-E Acetat, Hyaluronsäure und/oder deren Salze, Licochalcon A und/oder Pflanzenextrakte.

Das erfindungsgemäße Verfahren ist vorteilhaft dadurch gekennzeichnet, dass nach der Homogenisierung bei einer Temperatur von größer/gleich 80 °C die Zubereitung unter Rühren auf 70 bis 75 °C abgekühlt und anschließend in eine Gießform für Stifte vergossen und in dieser mit einer Abkühlungsrate von 0,2-0,4 °C/Sekunde auf Raumtemperatur abgekühlt wird.

Darüber hinaus ist es für das Verfahren erfindungsgemäß von Vorteil, wenn nach dem Abkühlen auf Raumtemperatur die stiftförmige Masse (Stiftmine) entformt und in ein Vorratsbehältnis für stiftförmige Zubereitungen umgefüllt wird.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **INCI** | **Beispiel A** | **Beispiel B** | **Beispiel C** | **Beispiel D** |
|---|---|---|---|---|---|
| Phase A | Behenyl Behenate | 5 | 4 | | 4 |
| | Prunus Amygdalus Dulcis Oil | 57,5 | 50,8 | 46,3 | 47,3 |
| | Cera Alba | | | 4 | |
| | Copernicia Cerifera Cera | 0,3 | 0,5 | 1,5 | 0,5 |
| | Ricinus Communis Seed Oil | 10 | 10 | 10 | 10 |
| | Helianthus Annuus Seed Cera | 1 | 2,5 | 2 | 2,5 |
| | Diisostearoyl Polyglyceryl-3 Dimer Dilinoleate | 1,4 | 1,4 | 1,4 | 1,4 |
| | Polyglyceryl-4 Diisostearate/Polyhydroxystearate/Sebacate | 0,8 | 0,8 | 0,8 | 0,8 |
| Phase B | Glycerin | 21 | 26 | 28 | 30 |
| | Aqua | 3 | 4 | 6 | 3,5 |
| | | 100 | 100 | 100 | 100 |

### Herstellung feste Formulierung:

- Phase A unter Rühren auf 90°C erhitzen
- Phase B unter Rühren auf 85°C erhitzen
- Phase B langsam zu Phase A geben, Rühren mit 800 - 1200 rpm
- Emulsion bei 80°C 20 Minuten nachrühren
- Anschließend bei 70-75°C in die Gießform geben
- Bei Raumtemperatur 15 Minuten auskühlen lassen. Die Abkühlgeschwindigkeit beträgt hierbei ca. 0,3°C/sek. Anschließend weitere 30 Minuten bei -18°C aushärten lassen
- Empfohlenes Equipment: IKA Rührwerk mit passendem Dissolver

### Herstellung Creme Formulierung:

- Phase A unter Rühren auf 90°C erhitzen
- Phase B unter Rühren auf 85°C erhitzen
- Phase B langsam zu Phase A geben, Rühren mit 150 rpm
- Heiße Emulsion homogenisieren
- Anschließend kalt Rühren (50-70 rpm)
- Emulsion bei 30°C nochmals homogenisieren

## Patentansprüche

1. Kosmetischer Lippenstift auf der Basis einer Glycerin-in-Öl-Emulsion enthaltend
a) Diisostearoyl Polyglyceryl-3 Dimer Dilinoleate,
b) einem zweiten Emulgator aus der Gruppe der Verbindungen Polyglyceryl-4 Diisostearate/Polyhydroxystearate/Sebacate,
c) Sonnenblumenwachs, wobei die Zubereitung frei ist von Mineralöl, Paraffinwachs, mikrokristallinem Wachs, Schellackwachs, Polyethylenwachsen, Polyacrylaten und quervernetzten Acrylat/C10-C30 Alkylacrylat Polymeren, **dadurch gekennzeichnet, dass** die Zubereitung Carnaubawachs enthält.

2. Verfahren zur Herstellung eines Lippenstiftes auf der Basis einer Glycerin-in-Öl-Emulsion, wobei die Ölphase
a) Diisostearoyl Polyglyceryl-3 Dimer Dilinoleate
b) Polyglyceryl-4 Diisostearate/Polyhydroxystearate/Sebacate,
c) Sonnenblumenwachs und
d) Carnaubawachs enthält, wobei die Zubereitung frei ist von Mineralöl, Paraffinwachs, mikrokristallinem Wachs, Schellackwachs, Polyethylenwachsen, Polyacrylaten und quervernetzten Acrylat/C10-C30 Alkylacrylat Polymeren, **dadurch gekennzeichnet, dass** die Glycerinphase und die Ölphase zunächst bei einer Temperatur von jeweils größer/gleich 80 °C vereinigt und homogenisiert werden.

3. Lippenstift nach Anspruch 1 oder Verfahren nach Anspruch 2, **dadurch gekennzeichnet, dass** die Zubereitung Glycerin in einer Menge von 21 bis 30 Gewichts-%, bezogen auf das Gesamtgewicht der Zubereitung, enthält.

4. Lippenstift oder Verfahren nach einem der vorhergehenden Ansprüche, **dadurch gekennzeichnet, dass** die Zubereitung Wasser in einer Konzentration weniger als 6 Gewichts-%, bezogen auf das Gesamtgewicht der Zubereitung, enthält.

5. Lippenstift oder Verfahren nach einem der vorhergehenden Ansprüche, **dadurch gekennzeichnet, dass** die Zubereitung Diisostearoyl Polyglyceryl-3 Dimer Dilinoleate in einer Konzentration von 1 bis 4 Gewichts-%, bezogen auf das Gesamtgewicht der Zubereitung, enthält.

6. Lippenstift oder Verfahren nach einem der vorhergehenden Ansprüche, **dadurch gekennzeichnet, dass** die Zubereitung Polyglyceryl-4 Diisostearate/Polyhydroxystearate/Sebacate in einer Konzentration von 0,5 bis 2,5 Gewichts-%, bezogen auf das Gesamtgewicht der Zubereitung, enthält.

7. Lippenstift oder Verfahren nach einem der vorhergehenden Ansprüche, **dadurch gekennzeichnet, dass** die Zubereitung Sonnenblumenwachs in einer Konzentration von 1,0 bis 10 Gewichts-%, bezogen auf das Gesamtgewicht der Zubereitung, enthält.

8. Lippenstift oder Verfahren nach einem der vorhergehenden Ansprüche, **dadurch gekennzeichnet, dass** die Zubereitung Carnaubawachs in einer Konzentration von 0,3 bis 2 Gewichts-%, bezogen auf das Gesamtgewicht der Zubereitung, enthält.

9. Lippenstift oder Verfahren nach einem der vorhergehenden Ansprüche, **dadurch gekennzeichnet, dass** die Zubereitung Behenylbehenat (INCI: Behenyl Behenate) enthält.

10. Lippenstift oder Verfahren nach einem der vorhergehenden Ansprüche, **dadurch gekennzeichnet, dass** die Zubereitung Behenylbehenat (INCI: Behenyl Behenate) in einer Konzentration von 1,0 bis 10 Gewichts-%, bezogen auf das Gesamtgewicht der Zubereitung, enthält.

11. Lippenstift oder Verfahren nach einem der vorhergehenden Ansprüche, **dadurch gekennzeichnet, dass** die Zubereitung Bienenwachs (INCI: Cera alba) enthält.

12. Lippenstift oder Verfahren nach einem der vorhergehenden Ansprüche, **dadurch gekennzeichnet, dass** die Zubereitung Bienenwachs (INCI: Cera alba) in einer Konzentration von 1 bis 10 Gewichts-%, bezogen auf das Gesamtgewicht der Zubereitung, enthält.

13. Verfahren nach einem der Ansprüche 2 bis 12, **dadurch gekennzeichnet, dass** nach der Homogenisierung bei einer Temperatur von größer/gleich 80 °C die Zubereitung unter Rühren auf 70 bis 75 °C abgekühlt und anschließend in eine Gießform für Stifte vergossen und in dieser mit einer Abkühlungsrate von 0,2-0,4 °C/Sekunde auf Raumtemperatur abgekühlt wird.

14. Verfahren nach Anspruch 13, **dadurch gekennzeichnet, dass** nach dem Abkühlen auf Raumtemperatur die stiftförmige Masse (Stiftmine) entformt und in ein Vorratsbehältnis für stiftförmige Zubereitungen umgefüllt wird.

## Claims

1. Cosmetic lipstick based on a glycerin-in-oil emulsion comprising
a) Diisostearoyl Polyglyceryl-3 Dimer Dilinoleate,
b) a second emulsifier from the group of compounds Polyglyceryl-4 Diisostearate/Polyhydroxystearate/Sebacate,
c) sunflower wax, the preparation being free of mineral oil, paraffin wax, microcrystalline wax, shellac wax, polyethylene waxes, polyacrylates and crosslinked acrylate/C10-C30 alkyl acrylate polymers, **characterized in that** the preparation comprises carnauba wax.

2. Process for producing a lipstick based on a glycerin-in-oil emulsion, the oil phase comprising
a) Diisostearoyl Polyglyceryl-3 Dimer Dilinoleate,
b) Polyglyceryl-4 Diisostearate/Polyhydroxystearate/Sebacate,
c) sunflower wax and
d) carnauba wax, the preparation being free of mineral oil, paraffin wax, microcrystalline wax, shellac wax, polyethylene waxes, polyacrylates and crosslinked acrylate/C10-C30 alkyl acrylate polymers, **characterized in that** the glycerin phase and the oil phase are first combined and homogenized at a temperature of in each case greater than/equal to 80°C.

3. Lipstick according to Claim 1 or process according to Claim 2, **characterized in that** the preparation comprises glycerin in an amount of 21% to 30% by weight, based on the total weight of the preparation.

4. Lipstick or process according to any of the preceding claims, **characterized in that** the preparation comprises water in a concentration of less than 6% by weight, based on the total weight of the preparation.

5. Lipstick or process according to any of the preceding claims, **characterized in that** the preparation comprises Diisostearoyl Polyglyceryl-3 Dimer Dilinoleate in a concentration of 1% to 4% by weight, based on the total weight of the preparation.

6. Lipstick or process according to any of the preceding claims, **characterized in that** the preparation comprises Polyglyceryl-4 Diisostearate/Polyhydroxystearate/Sebacate in a concentration of 0.5% to 2.5% by weight, based on the total weight of the preparation.

7. Lipstick or process according to any of the preceding claims, **characterized in that** the preparation comprises sunflower wax in a concentration of 1.0% to 10% by weight, based on the total weight of the preparation.

8. Lipstick or process according to any of the preceding claims, **characterized in that** the preparation comprises carnauba wax in a concentration of 0.3% to 2% by weight, based on the total weight of the preparation.

9. Lipstick or process according to any of the preceding claims, **characterized in that** the preparation comprises behenyl behenate (INCI: Behenyl Behenate).

10. Lipstick or process according to any of the preceding claims, **characterized in that** the preparation comprises behenyl behenate (INCI: Behenyl Behenate) in a concentration of 1.0% to 10% by weight, based on the total weight of the preparation.

11. Lipstick or process according to any of the preceding claims, **characterized in that** the preparation comprises beeswax (INCI: Cera Alba).

12. Lipstick or process according to any of the preceding claims, **characterized in that** the preparation comprises beeswax (INCI: Cera Alba) in a concentration of 1% to 10% by weight, based on the total weight of the preparation.

13. Process according to any of Claims 2 to 12, **characterized in that**, after the homogenization at a temperature of greater than/equal to 80°C, the preparation is cooled to 70°C to 75°C with stirring and then cast into a casting mould for sticks and cooled therein to room temperature at a cooling rate of 0.2-0.4°C/second.

14. Process according to Claim 13, **characterized in that**, after the cooling to room temperature, the stick-shaped compound (stick core) is demoulded and transferred into a storage container for stick-shaped preparations.

## Revendications

1. Rouge à lèvres cosmétique à base d'une émulsion glycérol-dans-huile, contenant
a) du dilinoléate de diisostéaroyl-polyglycéryl-3 dimère,
b) un deuxième émulsifiant du groupe des composés diisostéarate/polyhydroxystéarate/sébacate de polyglycéryl-4,
c) de la cire de tournesol, la préparation étant exempte d'huile minérale, de cire de paraffine, de cire microcristalline, de cire de gomme-laque, de cires de polyéthylène, de polyacrylates et de polymères réticulés d'acrylate/acrylate de C10-C30-alkyle, **caractérisé en ce que** la préparation contient de la cire de carnauba.

2. Procédé de préparation d'un rouge à lèvres à base d'une émulsion glycérol-dans-huile, la phase huileuse contenant
a) du dilinoléate de diisostéaroyl-polyglycéryl-3 dimère,
a) du diisostéarate/polyhydroxystéarate/sébacate de polyglycéryl-4 et
c) de la cire de tournesol et
d) de la cire de carnauba, la préparation étant exempte d'huile minérale, de cire de paraffine, de cire microcristalline, de cire de gomme-laque, de cires de polyéthylène, de polyacrylates et de polymères réticulés d'acrylate/acrylate de C10-C30-alkyle, **caractérisé en ce que** la phase de glycérol et la phase huileuse sont d'abord rassemblées à une température à chaque fois supérieure/égale à 80°C et homogénéisées.

3. Rouge à lèvres selon la revendication 1 ou procédé selon la revendication 2, **caractérisé en ce que** la préparation contient du glycérol en une quantité de 21 à 30% en poids par rapport au poids total de la préparation.

4. Rouge à lèvres ou procédé selon l'une des revendications précédentes, **caractérisé en ce que** la préparation contient de l'eau en une concentration inférieure à 6% en poids par rapport au poids total de la préparation.

5. Rouge à lèvres selon l'une des revendications précédentes, **caractérisé en ce que** la préparation contient du dilinoléate de diisostéaroyl-polyglycéryl-3 dimère en une concentration de 1 à 4% en poids par rapport au poids total de la préparation.

6. Rouge à lèvres ou procédé selon l'une des revendications précédentes, **caractérisé en ce que** la préparation contient du diisostéarate/polyhydroxystéarate/sébacate de polyglycéryl-4 en une concentration de 0,5 à 2,5% en poids par rapport au poids total de la préparation.

7. Rouge à lèvres ou procédé selon l'une des revendications précédentes, **caractérisé en ce que** la préparation contient de la cire de tournesol en une concentration de 1,0 à 10% en poids par rapport au poids total de la préparation.

8. Rouge à lèvres ou procédé selon l'une des revendications précédentes, **caractérisé en ce que** la préparation contient de la cire de carnauba en une concentration de 0,3 à 2% en poids par rapport au poids total de la préparation.

9. Rouge à lèvres ou procédé selon l'une des revendications précédentes, **caractérisé en ce que** la préparation contient du béhénate de béhényle (INCI : Behenyl Behenate).

10. Rouge à lèvres ou procédé selon l'une des revendications précédentes, **caractérisé en ce que** la préparation contient du béhénate de béhényle (INCI : Behenyl Behenate) en une concentration de 1,0 à 10% en poids par rapport au poids total de la préparation.

11. Rouge à lèvres ou procédé selon l'une des revendications précédentes, **caractérisé en ce que** la préparation contient de la cire d'abeille (INCI : Cera alba).

12. Rouge à lèvres ou procédé selon l'une des revendications précédentes, **caractérisé en ce que** la préparation contient de la cire d'abeille (INCI : Cera alba) en une concentration de 1 à 10% en poids par rapport au poids total de la préparation.

13. Procédé selon l'une des revendications 2 à 12, **caractérisé en ce qu'**après l'homogénéisation à une température supérieure ou égale à 80°C, la préparation est refroidie sous agitation à 70 jusqu'à 75°C, puis coulée dans un moule pour crayons et refroidie dans celui-ci à une vitesse de refroidissement de 0,2-0,4°C/s jusqu'à température ambiante.

14. Procédé selon la revendication 13, **caractérisé en ce qu'**après refroidissement à température ambiante, la masse en forme de crayon (mine de crayon) est démoulée et transférée dans un récipient de stockage pour préparations en forme de crayons.
